# EUROPEAN PATENT APPLICATION

(11) **EP 4 755 379 A1**
(43) Date of publication of application: **10.06.2026**
(21) Application number: 24851177.6
(22) Date of filing: 02.08.2024
(51) Int. Cl.: A61K 31/047, A61K 33/24, A61P 3/10

(54) **CHROMIUM-INOSITOL COMPOSITION**

(30) Priority: 04.08.2023 ES 202330679
(71) Applicant: ITF Research Pharma, S.L.U., 28108 Alcobendas, Madrid (ES)
(72) Inventor: URSO, Katia, 28108 Alcobendas, Madrid (ES); ESQUINAS GONZÁLEZ, Pedro Enrique, 28108 Alcobendas, Madrid (ES)
(74) Representative: ABG Intellectual Property Law, S.L.
(86) International application number: PCT/ES2024/070494
(87) International publication number: WO 2025/032275

(57) **Abstract**

The present invention relates to compositions comprising inositol, chromium and, optionally, vitamin D. The compositions of the invention are characterised by their low relative amount of chromium and they can be used as nutritional supplement or drug for the treatment and/or the prevention of a metabolic disease and/or disorder characterised by elevated plasma glucose levels.

## Description

### FIELD OF THE INVENTION

The present invention relates to a composition comprising inositol and chromium, to its preparation and to the use thereof in the treatment of diabetes mellitus, particularly gestational diabetes mellitus.

### STATE OF THE ART

Diabetes mellitus (DM) is a group of metabolic disorders the common characteristic of which is the existence of persistent or chronic elevated plasma glucose (blood sugar) levels. The cause may be a defect in insulin production, resistance to the action of this hormone, an increase in glucose production, or a combination thereof. These high glucose levels are often accompanied by abnormalities in the metabolism of lipids, proteins, mineral salts and electrolytes.

DM is associated with the occurrence of complications in many organ systems, with the most evident being vision loss (diabetic retinopathy), progressive functional deterioration of kidneys (diabetic nephropathy), involvement of blood vessels (diabetic vasculopathy) and the heart (coronary heart disease and acute myocardial infarction), cerebral involvement and intestinal irrigation; however, the most prevalent complications affect the peripheral and autonomic nervous system.

Gestational diabetes mellitus (GDM) is defined as a state of glucose intolerance in pregnant women without prior diagnosis of diabetes. It affects about 10% of pregnant women worldwide. GDM is the result of an interaction between genetic and environmental factors; among the most notable risk factors are maternal age, increased body mass index, ethnicity, family history of type 2 diabetes, and previous history of GDM.

GDM is associated with a higher risk of pregnancy complications, such as gestational hypertension, macrosomy, shoulder dystocia, preterm birth, caesarean delivery, neonatal hypoglycaemia, and increased perinatal mortality. Furthermore, it predisposes women and their offspring to the development of type 2 diabetes (Teh, WT et al. (2011). Risk factors for gestational diabetes mellitus: Implications for the application of screening guidelines. Australian and New Zealand Journal of Obstetrics and Gynaecology 2011, 51, 26-30).

Although some people suffering from diabetes must take drugs to control glucose levels, other people with type 2 diabetes can control their blood glucose level with a suitable meal plan and physical activity. Likewise, treatment of gestational diabetes begins with nutritional control, physical exercise and self-monitoring of blood glucose.

In this sense, food supplements may play an important role in the treatment, management and/or prevention of diabetes, particularly gestational diabetes.

Several studies have demonstrated that there is an inverse relationship between serum glucose levels and dietary intake of some nutrients. The composition of the present invention aims to provide nutrients that may contribute to controlling plasma glucose levels and can therefore be useful in the prevention and/or treatment of diabetes, particularly gestational diabetes.

Inositol (cyclohexane-1,2,3,4,5,6-hexol) is a carbocycle of the polyol family. It exists in nine stereoisomeric forms as a result of epimerisation of their hydroxyl groups, with myo-inositol being the most common isomer in animal tissues and natural foods.

It is part of all cell membranes and has the function of participating in lipid metabolism, as well as contributing to the functioning of muscles and nerves. It is also characterised by its insulin-mimetic properties.

Chromium is a mineral that intervenes in glucose homeostasis by acting as an insulin cofactor. Its deficiency has been observed to have a significant effect on glucose intolerance. Some research showed that plasma chromium concentrations were substantially lower than normal during pregnancy and this may represent normal physiology as well as depletion of maternal reserves (Woods, S.E. et al. (2008). Serum Chromium and Gestational Diabetes. Journal of the American Board of Family Medicine (JABFM) March-April 2008, 21(2), 153-157).

Some food supplements for the prevention of GDM are already known. However, many of these food supplements do not have a well-established efficacy, so effective supplements are needed for the treatment and/or the prevention of GDM.

### SUMMARY OF THE INVENTION

The inventors have found that a composition comprising inositol and chromium, preferably in a chromium/inositol weight ratio between 1/100,000 and 1/800,000, can be used in the treatment and/or the prevention of diabetes, particularly gestational diabetes mellitus.

Therefore, a first aspect of the present invention relates to a composition comprising inositol, in the form of at least one of its pharmaceutically acceptable isomers; and chromium, preferably in the form of a pharmaceutically acceptable chromium compound.

A second aspect of the present invention relates to a composition according to the invention for use in the treatment and/or the prevention of a metabolic disease and/or disorder characterised by the existence of elevated plasma glucose levels, for example, diabetes mellitus, particularly gestational diabetes mellitus.

A third aspect relates to the method for manufacturing the composition of the present invention.

### DETAILED DESCRIPTION

In a first aspect, the present invention relates to a composition comprising inositol and chromium.

In one embodiment, inositol refers to one or more of the inositol isomers. The term "inositol isomers" refers to inositol stereoisomers which have, in particular, the following structures:

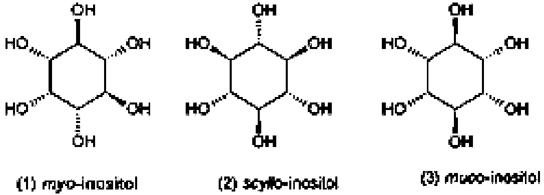

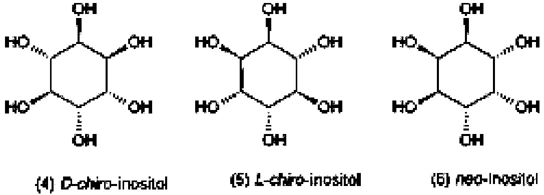

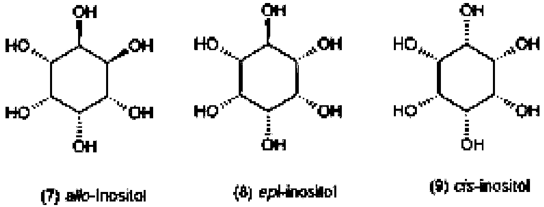

In a preferred embodiment, the inositol is myo-inositol.

In another preferred embodiment, the inositol is a mixture of inositol isomers comprising myo-inositol, preferably as the isomer in the highest weight percentage.

In a preferred embodiment, the inositol is a mixture of inositol isomers comprising myo-inositol, preferably as the isomer in the highest weight percentage, and another one of the inositol isomers, wherein preferably said another isomer is D-chiro-inositol or scyllo-inositol. In a particular embodiment, said another isomer is D-chiro-inositol. In another particular embodiment, said another isomer is scyllo-inositol.

In a more particular embodiment, the inositol is a mixture of inositol isomers comprising myo-inositol, preferably as the isomer in the highest weight percentage, D-chiro-inositol and scyllo-inositol.

In a preferred embodiment, the chromium is chromium III.

In a preferred embodiment, in any embodiment described herein, the chromium, particularly chromium III, is present in the form of a chromium compound.

The term "chromium compound" refers to any pharmaceutically acceptable compound comprising chromium, preferably any pharmaceutically acceptable compound comprising chromium III.

The term "pharmaceutically acceptable" indicates that the ingredient it describes is physiologically tolerated, which means that it is not toxic when suitably used for its therapeutic purpose, particularly when used at reasonable medical doses in a treatment according to the present invention.

In a preferred embodiment, the pharmaceutically acceptable chromium compound, preferably pharmaceutically acceptable chromium III compound, is a pharmaceutically acceptable chromium salt or chelate, preferably a pharmaceutically acceptable chromium chelate.

Non-limiting examples of pharmaceutically acceptable chromium salts are chromium chloride, chromium sulphate or chromium nitrate.

Non-limiting examples of pharmaceutically acceptable chromium chelates are chromium nicotinate, chromium lactate trihydrate, chromium citrate or chromium picolinate.

In a preferred embodiment, the chromium compound is chromium (III) picolinate, a coordination complex of formula:

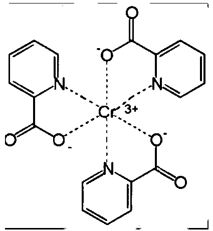

In a preferred embodiment, the weight ratio between chromium and inositol in any of the compositions of the present invention is comprised between 1/100,000 and 1/800,000, preferably between 1/104,000 and 1/720,000, of chromium/inositol.

In a particularly preferred embodiment, the composition of the present invention comprises the pharmaceutically acceptable chromium compound, which is chromium picolinate; and the inositol, which is myo-inositol or a mixture of inositol isomers comprising myo-inositol as the isomer in the highest weight percentage, as defined in any preceding embodiment, more preferably the inositol is myo-inositol.

Preferably, the weight ratio between chromium picolinate and myo-inositol in any of these compositions is comprised between 1/12,500 and 1/100,000, preferably between 1/13,000 and 1/90,000, chromium picolinate/myo-inositol.

This weight ratio between the components allows having, in the same formulation, therapeutic doses of myo-inositol (2 to 4 g of inositol) with minimal therapeutic amounts (EFSA Journal, 8(10):1732, 23 pp) of chromium, ensuring at the same time a minimal exposure to this metal in the case of administration to pregnant women (for the prevention and/or the treatment of GDM). In this way, the recommended amounts of both active ingredients can be achieved with the administration of a smaller number of daily doses (preferably one or two doses a day), contributing to compliance with treatment.

Additionally, this association of inositol and chromium has a synergistic effect that allows reducing the relative amount of chromium in the formulation without compromising efficacy.

In a preferred embodiment, the composition of the invention may contain additional active ingredients. Particularly, it may contain vitamins, preferably vitamin D and/or one or more group B vitamins) and/or minerals, for example, iron or a pharmaceutically acceptable compound containing same, and/or zinc or a pharmaceutically acceptable compound containing same. Preferably, the composition contains vitamin D, more preferably vitamin D3 (also known as cholecalciferol). Even more preferably, the composition of the present invention contains vitamin D3 formulated with excipients that allow its dispersion in cold water. Particularly, it contains vitamin D3 marketed by DSM under the name *Dry Vitamin D3 100* CWS (reference 5012015), i.e., a composition comprising:
- granules comprising:
   ∘ a mixture of vitamin D3, preferably Dry Vitamin D3, and edible fats, said mixture preferably being dispersed in a matrix,
   ∘ wherein the matrix comprises a sugar, preferably sucrose, and gelatine, preferably hydrolysed bovine gelatine;
   ∘ wherein the matrix is coated with a starch, preferably cornstarch;
- optionally, an antioxidant, preferably tocopherol, more specifically dl-α-tocopherol;
- optionally, a glidant, preferably silicon dioxide;
wherein:
- more than 90%, preferably 100%, of the granules, go through a sieve with a pore diameter of 850 µm;
- more than 80%, preferably more than 90%, of the granules, go through a sieve with a pore diameter of 500 µm;
- less than 25%, preferably less than 15%, of the granules, go through a sieve with a pore diameter of 150 µm; and
wherein this composition comprises 100,000 - 110,000 IU (or 2.5 - 2.75 mg) of vitamin D3 per gram of this composition.

The term "edible fats" refers to animal or plant fats comprising fatty acid glycerides as the constituent in the highest amount by weight.

The term "dry" refers to the ingredient it describes as comprising less than 10% by weight, preferably less than 1% by weight, more preferably less than 0.1% by weight, of water.

The composition of the present invention can be administered once a week, every three days, every two days, once a day, two times a day, three times a day or four times a day, preferably one or two times a day.

In addition or as an alternative to the weight ratio between the chromium or chromium compound and inositol, the composition of the present invention can be characterised by the specific amounts of the different ingredients of the composition.

In a preferred embodiment, the composition of the present invention comprises between 600 and 6,000 mg, particularly between 1,000 and 6,000 mg, more particularly between 1,000 and 5,000 mg, of inositol, preferably myo-inositol or a mixture of inositol stereoisomers which contains myo-inositol in a majority percentage by weight, as defined above.

In a particular embodiment, the composition of the present invention comprises between 3,000 and 5,000 mg, still more particularly between 3,500 and 4,500 mg, of inositol, preferably myo-inositol or a mixture of inositol stereoisomers which contains myo-inositol in a majority percentage by weight, as defined above. These amounts are particularly suitable when the composition is in the form of a granulate or powder contained in a sachet or in the form of a granulate or powder dissolved or dispersed in a pharmaceutically acceptable liquid such as water, preferably for oral administration.

In another particular embodiment, the composition of the present invention comprises between 1,000 and 3,000 mg, particularly between 1,500 and 2,500 mg, of inositol, preferably myo-inositol or a mixture of inositol stereoisomers which contains myo-inositol in a majority percentage by weight, as defined above.

In another particular embodiment, the composition of the present invention comprises between 600 and 1,500 mg of inositol, preferably myo-inositol or a mixture of inositol stereoisomers which contains myo-inositol in a majority percentage by weight, as defined above. These amounts are particularly suitable when the composition is in the form of a capsule or tablet for oral administration.

In a preferred embodiment, the composition of the present invention comprises between 2.5 and 250 µg, particularly between 6 and 125 µg, more particularly between 8.5 and 62.5 µg of chromium, preferably chromium III, more preferably between 20 and 2,000 µg, particularly between 50 and 1,000 µg, more particularly between 70 and 500 µg, of chromium picolinate.

In another particular embodiment, the composition of the present invention comprises between 2.5 and 62.5 µg, particularly between 3.1 and 31.0 µg, of chromium, preferably chromium III, more preferably between 20 and 500 µg, particularly between 25 and 250 µg, of chromium picolinate.

In another particular embodiment, the composition of the present invention comprises between 1.2 and 12.5 µg of chromium, preferably chromium III, more preferably between 10 and 100 µg of chromium picolinate.

In a preferred embodiment, the composition of the present invention comprises:
- between 600 and 6,000 mg, for example between 1,000 and 6,000 mg, of inositol, preferably myo-inositol or a mixture of inositol stereoisomers which contains myo-inositol in a majority percentage by weight,
- between 2.5 and 250 µg of chromium, preferably chromium III, more preferably between 20 and 2,000 µg of a chromium III compound which is chromium picolinate,
- optionally, between 5 and 100 µg of vitamin D (corresponding to between 200 and 4,000 IU), preferably vitamin D3.

Preferably, the composition of the present invention has a weight ratio between chromium picolinate and myo-inositol comprised between 1/12,500 and 1/100,000, preferably between 1/13,000 and 1/90,000 of chromium picolinate/myo-inositol. In other words, the composition of the present invention has a weight ratio between chromium, or chromium III, and inositol, or myo-inositol, comprised between 1/100,000 and 1/800,000, preferably between 1/104,000 and 1/720,000, of chromium/inositol.

In a particularly preferred embodiment, the composition of the present invention comprises:
- between 600 and 6,000 mg, for example between 1,000 and 6,000 mg, of inositol, preferably myo-inositol or a mixture of inositol stereoisomers which contains myo-inositol in a majority percentage by weight,
- between 2.5 and 250 µg of chromium, preferably chromium III, more preferably between 20 and 2,000 µg of a chromium III compound which is chromium picolinate,
- optionally, between 5 and 100 µg of vitamin D (corresponding to between 200 and 4,000 IU), preferably vitamin D3,
wherein the weight ratio between chromium, or chromium III, and inositol, or myo-inositol, is comprised between 1/100,000 and 1/800,000, preferably between 1/104,000 and 1/720,000, of chromium/inositol. Alternatively, when it contains chromium picolinate, the weight ratio between chromium picolinate and myo-inositol is comprised between 1/12,500 and 1/100,000, preferably between 1/13,000 and 1/90,000, of chromium picolinate/myo-inositol.

In a preferred embodiment, the composition of the present invention comprises:
- between 1,000 and 5,000 mg of inositol, preferably myo-inositol or a mixture of inositol stereoisomers which contains myo-inositol in a majority percentage by weight,
- between 2.5 and 250 µg of chromium, preferably chromium III, more preferably between 20 and 2,000 µg a chromium III compound which is chromium picolinate,
- optionally, between 5 and 100 µg of vitamin D (corresponding to between 200 and 4,000 IU), preferably vitamin D3.

Preferably, the composition of the present invention has a weight ratio between chromium picolinate and myo-inositol comprised between 1/12,500 and 1/100,000, preferably between 1/13,000 and 1/90,000, more preferably between 1/15,000 and 1/90,000, even more preferably between 1/20,000 and 1/75,000, between 1/23,000 and 1/70,000, between 1/25,000 and 1/63,000, particularly about 1/50,000, of chromium picolinate/myo-inositol. In other words, the composition of the present invention has a weight ratio between chromium, or chromium III, and inositol, or myo-inositol, comprised between 1/100,000 and 1/800,000, preferably between 1/104,000 and 1/720,000, more preferably between 1/120,000 and 1/720,000, between 1/160,000 and 1/600,000, between 1/184,000 and 1/560,000, between 1/200,000 and 1/504,000, particularly 1/400,000, of chromium/inositol.

In a particularly preferred embodiment, the composition of the present invention comprises:
- between 1,000 and 5,000 mg of inositol, preferably myo-inositol or a mixture of inositol stereoisomers which contains myo-inositol in a majority percentage by weight,
- between 2.5 and 250 µg of chromium, preferably chromium III, more preferably between 20 and 2,000 µg of a chromium III compound which is chromium picolinate,
- optionally, between 5 and 100 µg of vitamin D (corresponding to between 200 and 4,000 IU), preferably vitamin D3,
wherein the weight ratio between chromium, or chromium III, and inositol, or myo-inositol, is comprised between 1/100,000 and 1/800,000, preferably between 1/104,000 and 1/720,000, more preferably between 1/120,000 and 1/720,000, between 1/160,000 and 1/600,000, between 1/184,000 and 1/560,000, between 1/200,000 and 1/504,000, particularly 1/400,000, of chromium/inositol.

Alternatively, when it contains chromium picolinate, the weight ratio between chromium picolinate and myo-inositol is comprised between 1/12,500 and 1/100,000, preferably between 1/13,000 and 1/90,000, more preferably between 1/15,000 and 1/90,000, even more preferably between 1/20,000 and 1/75,000, between 1/23,000 and 1/70,000, between 1/25,000 and 1/63,000, particularly about 1/50,000, of chromium picolinate/myo-inositol.

In a preferred embodiment, the composition comprises:
- between 3,000 and 5,000 mg of inositol, preferably myo-inositol or a mixture of inositol stereoisomers which contains myo-inositol in a majority percentage by weight,
- between 6 and 125 µg of chromium, preferably chromium III, more preferably between 50 and 1,000 µg of a chromium III compound which is chromium picolinate,
- optionally, between 15 and 100 µg of vitamin D, preferably vitamin D3.

This embodiment corresponds with a unit dose when the composition of the invention is to be administered once a day.

These amounts are particularly suitable when the composition is in the form of a granulate or powder contained in a sachet or in the form of a granulate or powder dissolved or dispersed in a pharmaceutically acceptable liquid such as water, preferably for oral administration.

Preferably, the composition of the present invention has a weight ratio between chromium picolinate and myo-inositol of the composition comprised between 1/15,000 and 1/90,000, preferably between 1/20,000 and 1/75,000, more preferably between 1/23,000 and 1/70,000, even more preferably between 1/25,000 and 1/63,000, particularly about 1/50,000, of chromium picolinate/myo-inositol. In other words, the composition of the present invention has a weight ratio between chromium, or chromium III, and inositol, or myo-inositol, comprised between 1/120,000 and 1/720,000, preferably between 1/160,000 and 1/600,000, more preferably between 1/184,000 and 1/560,000, even more preferably between 1/200,000 and 1/504,000, particularly 1/400,000, of chromium/inositol.

In a particularly preferred embodiment, the composition comprises:
- between 3,000 and 5,000 mg of inositol, preferably myo-inositol or a mixture of inositol stereoisomers which contains myo-inositol in a majority percentage by weight,
- between 6 and 125 µg of chromium, preferably chromium III, more preferably between 50 and 1,000 µg of a chromium III compound which is chromium picolinate,
- optionally, between 15 and 100 µg of vitamin D, preferably vitamin D3,
wherein the weight ratio between chromium, or chromium III, and inositol, or myo-inositol, is comprised between 1/120,000 and 1/720,000, preferably between 1/160,000 and 1/600,000, more preferably between 1/184,000 and 1/560,000, even more preferably between 1/200,000 and 1/504,000, particularly 1/400,000 of chromium/inositol.

Alternatively, when it contains chromium picolinate, the weight ratio between chromium picolinate and myo-inositol is comprised between 1/15,000 and 1/90,000, preferably between 1/20,000 and 1/75,000, more preferably between 1/23,000 and 1/70,000, even more preferably between 1/25,000 and 1/63,000, particularly about 1/50,000, of chromium picolinate/myo-inositol.

This embodiment corresponds with a unit dose when the composition of the invention is to be administered once a day.

In a particularly preferred embodiment, the composition comprises:
- between 3,500 and 4,500 mg of inositol, preferably myo-inositol or a mixture of inositol stereoisomers which contains myo-inositol in a majority percentage by weight ,
- between 8.5 and 62.5 µg of chromium, preferably chromium III, more preferably between 70 and 500 µg of a chromium III compound which is chromium picolinate,
- optionally, between 20 and 50 µg of vitamin D, preferably vitamin D3.

Preferably, the weight ratio between chromium picolinate and myo-inositol is comprised between 1/20,000 and 1/75,000, preferably between 1/23,000 and 1/70,000, more preferably 1/25,000 and 1/63,000, particularly about 1/50,000, of chromium picolinate/myo-inositol. In other words, the composition of the present invention has a weight ratio between chromium, or chromium III, and inositol, or myo-inositol, comprised between 1/184,000 and 1/560,000, preferably between 1/200,000 and 1/504,000, particularly 1/400,000, of chromium/inositol.

In a particularly preferred embodiment, the composition comprises:
- between 3,500 and 4,500 mg of inositol, preferably myo-inositol or a mixture of inositol stereoisomers which contains myo-inositol in a majority percentage by weight,
- between 8.5 and 62.5 µg of chromium, preferably chromium III, more preferably between 70 and 500 µg of chromium picolinate,
- optionally, between 20 and 50 µg of vitamin D, preferably vitamin D3,
wherein the weight ratio between chromium, or chromium III, and inositol, or myo-inositol, is comprised between 1/184,000 and 1/560,000, preferably between 1/200,000 and 1/504,000, particularly 1/400,000, of chromium/inositol.

Alternatively, when it contains chromium picolinate, the weight ratio between chromium picolinate and myo-inositol is comprised between 1/20,000 and 1/75,000, preferably between 1/23,000 and 1/70,000, more preferably between 1/25,000 and 1/63,000, particularly about 1/50,000, of chromium picolinate/myo-inositol.

This embodiment corresponds with a unit dose when the composition of the invention is to be administered once a day.

In a preferred embodiment, the composition of the invention comprises:
- between 1,000 and 3,000 mg of inositol, preferably myo-inositol or a mixture of inositol stereoisomers which contains myo-inositol in a majority percentage by weight,
- between 2.5 and 62.5 µg of chromium, preferably chromium III, more preferably between 20 and 500 µg of a chromium III compound which is chromium picolinate,
- optionally, between 5 and 50 µg of vitamin D, preferably vitamin D3.

This embodiment corresponds with a unit dose when the composition of the invention is to be administered two times a day.

These amounts are particularly suitable when the composition is in the form of a granulate or powder contained in a sachet or in the form of a granulate or powder dissolved or dispersed in a pharmaceutically acceptable liquid such as water, preferably for oral administration.

Preferably, the weight ratio between chromium picolinate and myo-inositol of the composition is comprised between 1/15,000 and 1/100,000, preferably between 1/20,000 and 1/75,000, more preferably between 1/23,000 and 1/70,000, even more preferably between 1/25,000 and 1/63,000, particularly about 1/50,000, of chromium picolinate/myo-inositol. In other words, the weight ratio between chromium, or chromium III, and inositol, or myo-inositol, is comprised between 1/120,000 and 1/800,000, preferably between 1/160,000 and 1/600,000, more preferably between 1/184,000 and 1/560,000, even more preferably between 1/200,000 and 1/504,000, particularly 1/400,000, of chromium/inositol.

In a particularly preferred embodiment, the composition of the invention comprises:
- between 1,000 and 3,000 mg of inositol, preferably myo-inositol or a mixture of inositol stereoisomers which contains myo-inositol in a majority percentage by weight,
- between 2.5 and 62.5 µg of chromium, preferably chromium III, more preferably between 20 and 500 µg of chromium picolinate,
- optionally, between 5 and 50 µg of vitamin D, preferably vitamin D3,
wherein the weight ratio between chromium, or chromium III, and inositol, or myo-inositol, is comprised between 1/120,000 and 1/800,000, preferably between 1/160,000 and 1/600,000, more preferably between 1/184,000 and 1/560,000, even more preferably between 1/200,000 and 1/504,000, particularly 1/400,000, of chromium/inositol.

Alternatively, when it contains chromium picolinate, the weight ratio between chromium picolinate and myo-inositol is comprised between 1/15,000 and 1/100,000, preferably between 1/20,000 and 1/75,000, more preferably between 1/23,000 and 1/70,000, even more preferably between 1/25,000 and 1/63,000, particularly about 1/50,000, of chromium picolinate/myo-inositol.

This embodiment corresponds with a unit dose when the composition of the invention is to be administered two times a day.

In a particularly preferred embodiment, the composition comprises:
- between 1,500 and 2,500 mg of inositol, preferably myo-inositol or a mixture of inositol stereoisomers which contains myo-inositol in a majority percentage by weight,
- between 3.1 and 31.0 µg of chromium, preferably chromium III, more preferably between 25 and 250 µg of a chromium III compound which is chromium picolinate,
- optionally, between 10 and 25 µg of vitamin D, preferably vitamin D3.

Preferably, the composition of the present invention has a weight ratio between chromium picolinate and myo-inositol comprised between 1/20,000 and 1/75,000, preferably between 1/23,000 and 1/70,000, more preferably between 1/25,000 and 1/63,000, particularly about 1/50,000, of chromium picolinate/myo-inositol. In other words, the composition of the present invention has a weight ratio between chromium, or chromium III, and inositol, or myo-inositol, comprised between 1/184,000 and 1/560,000, preferably between 1/200,000 and 1/504,000, particularly 1/400,000, of chromium/inositol.

In a particularly preferred embodiment, the composition comprises:
- between 1,500 and 2,500 mg of inositol, preferably myo-inositol or a mixture of inositol stereoisomers which contains myo-inositol in a majority percentage by weight,
- between 3.1 and 31.0 µg of chromium, preferably chromium III, more preferably between 25 and 250 µg of a chromium III compound which is chromium picolinate,
- optionally, between 10 and 25 µg of vitamin D, particularly of vitamin D3,
wherein the weight ratio between chromium, or chromium III, and inositol, or myo-inositol, is comprised between 1/184,000 and 1/560,000, preferably between 1/200,000 and 1/504,000, particularly 1/400,000, of chromium/inositol.

Alternatively, when it contains chromium picolinate, the weight ratio between chromium picolinate and myo-inositol is comprised between 1/20,000 and 1/75,000, preferably between 1/23,000 and 1/70,000, more preferably between 1/25,000 and 1/63,000, particularly about 1/50,000, of chromium picolinate/myo-inositol.

This embodiment corresponds with a unit dose when the composition of the invention is to be administered two times a day.

Alternatively, the composition of the invention comprises:
- between 600 and 1,500 mg of inositol, preferably myo-inositol or of a mixture of inositol stereoisomers which contains myo-inositol in a majority percentage by weight,
- between 1.2 and 12.5 µg of chromium, preferably chromium III, more preferably between 10 and 100 µg of a chromium III compound which is chromium picolinate,
- optionally, between 5 and 50 µg of vitamin D, preferably vitamin D3.

This embodiment corresponds with a unit dose when the composition of the invention is to be administered from two times a day to four times a day, preferably two or three times a day.

These amounts are particularly suitable when the composition is in the form of a capsule or tablet for oral administration.

Preferably, the composition of the present invention has a weight ratio between chromium picolinate and myo-inositol comprised between 1/13,000 and 1/75,000, preferably between 1/15,000 and 1/50,000, of chromium picolinate/myo-inositol. In other words, the composition of the present invention has a weight ratio between chromium, or chromium III, and inositol, or myo-inositol, comprised between 1/104,000 and 1/600,000, more preferably between 120,000 and 1/400,000, of chromium/inositol.

In another preferred embodiment the composition of the invention comprises:
- between 600 and 1,500 mg of inositol, preferably myo-inositol or of a mixture of inositol stereoisomers which contains myo-inositol in a majority percentage by weight,
- between 1.2 and 12.5 µg of chromium, preferably chromium III, more preferably between 10 and 100 µg of a chromium III compound which is chromium picolinate,
- optionally, between 5 and 50 µg of vitamin D, preferably vitamin D3,
wherein the weight ratio between chromium, or chromium III, and inositol, or myo-inositol, is comprised between 1/104,000 and 1/600,000, particularly between 1/120,000 and 1/400,000, of chromium/inositol.

Alternatively, when it contains chromium picolinate, the weight ratio between chromium picolinate and myo-inositol is comprised between 1/13,000 and 1/75,000, preferably between 1/15,000 and 1/50,000, of chromium picolinate/myo-inositol.

This embodiment corresponds with a unit dose when the composition of the invention is to be administered from two times a day to four times a day, preferably two or three times a day.

The term "majority" or "in a majority percentage" refers to the part of a whole that represents the largest percentage of the whole. Particularly, in the context of the present invention, when it is indicated that myo-inositol is present in a majority percentage by weight in a mixture of stereoisomers, this means that myo-inositol is present in an amount greater than 50% by weight of the mixture, preferably greater than 60%, and more preferably even greater than 70%.

The terms "around" or "about" refer to a slight variation from the specified value, preferably within 10 percent of the specified value. However, the terms "around" or "about" may mean a greater tolerance of variation depending, for example, on the experimental technique used. Said variations from a specified value are known to person skilled in the art and are within the context of the present invention.

In addition or as an alternative to the weight ratio between the chromium or chromium compound and inositol, and/or to the specific amounts of the different ingredients of the composition, the composition of the present invention can be characterised by its particle size distribution.

In a preferred embodiment, the inositol and chromium, preferably in the form of a chromium compound, used to prepare the composition of the invention, and therefore also the inositol and chromium, preferably in the form of a chromium compound, comprised in the composition of the invention, have a similar particle size distribution.

Particularly:
- the inositol, preferably myo-inositol or a mixture of inositol stereoisomers which contains myo-inositol in a majority percentage by weight, has a (volume weighted) particle size between 0.5 and 600 µm, preferably at least 90% of the particles have a (volume weighted) particle size between 1 and 250 µm;
   and
- the chromium, preferably a chromium compound, more preferably chromium picolinate, has a (volume weighted) particle size between 0.1 and 200 µm, preferably at least 90% of the particles have a (volume weighted) particle size between 1 and 100 µm.

Particularly:
- the inositol, preferably myo-inositol or a mixture of inositol stereoisomers which contains myo-inositol in a majority percentage by weight, has a particle size distribution D90 equal to or less than 150 µm, such as between 150 and 70 µm, preferably between 100 and 120, more preferably between 105 and 115 µm, particularly about 111 µm;
   and
- the chromium, preferably a chromium compound, more preferably chromium picolinate, has a particle size distribution D90 equal to or less than 75 µm, such as between 75 and 35 µm, preferably between 65 and 45 µm, particularly about 53 µm.;

Particularly:
- the inositol, preferably myo-inositol or a mixture of inositol stereoisomers which contains myo-inositol in a majority percentage by weight, has a particle size distribution D50 equal to or less than 30 µm, such as between 30 and 10 µm, preferably between 25 and 15 µm, particularly about 22 µm;
   and
- the chromium, preferably a chromium compound, more preferably chromium picolinate, has a particle size distribution D50 equal to or less than 30 µm, such as between 30 and 10 µm, preferably between 20 and 10 µm, particularly about 16 µm.

Particularly:
- the inositol, preferably myo-inositol or a mixture of inositol stereoisomers which contains myo-inositol in a majority percentage by weight, has a particle size distribution D10 equal to or less than 5 µm, such as between 5 and 1 µm, preferably between 4 and 2 µm, particularly about 3 µm;
   and
- the chromium, preferably a chromium compound, more preferably chromium picolinate, has a particle size distribution D10 equal to or less than 5 µm, such as between 5 and 1 µm, preferably between 4 and 2 µm, particularly about 3 µm.

The expression "DX equal to or less than" is known to one skilled in the art and refers to the particle size distribution of a sample, indicating that around X% by volume of all the particles of the sample have a (volume weighted) size equal to or less than the indicated figure. For example, in the context of the present invention, D90 equal to or less than 150 µm indicates that 90% by volume of the particles have a size equal to or less than 150 µm.

The value of the particle size and particle size distribution DX can be measured, for example, by means of laser diffraction methods according to the ISO 13320:2020 standard, Edition 2, 2020-01.

For example, particle size and particle size distribution DX can be measured by means of laser diffraction, for example, using the equipment Mastersizer 2000 of Malvern Instruments (Accessory name: Sirocco 2000, Analysis model: General purpose), and more particularly selecting the parameters:
- Particle RI: 1.520;
- Absorption: 0.1;
- Dispersant RI: 1,000;
- Size range: 0.020 to 2000.000 µm;
- Weighted residual: 0.104-0.132%
- Sensitivity: Enhanced;
- Obscuration: 3.04-3.68%;

Particularly,
- the inositol, preferably myo-inositol or a mixture of inositol stereoisomers which contains myo-inositol in a majority percentage by weight, has a (volume weighted) mean particle diameter D[4,3] between 40 and 50 µm, preferably about 45 µm,
   and
- the chromium, preferably a chromium compound, more preferably chromium picolinate, has a (volume weighted) mean particle diameter D[4,3] between 20 and 30 µm, preferably about 24 µm.

The term "D[4,3]", also referred to as "De Brouckere diameter" is known to one skilled in the art and indicates the volume weighted mean particle size distribution.

The value of diameter D[4,3] can be measured in the same manner as indicated for the value of the particle size and particle size distribution DX.

Preferably, the ratio between the mean particle diameters D[4,3] of the inositol, preferably myo-inositol or a mixture of inositol stereoisomers which contains myo-inositol in a majority percentage by weight, and of the chromium, preferably a chromium compound, more preferably chromium picolinate, is between 3/1 and 1/1, preferably between 2.5/1 and 1.5/1.

Surprisingly, this similar particle size distribution, given by the aforementioned ratio of the diameters D[4,3] of the inositol and chromium or chromium compound particles, contributes to the suitable mixing of the components of the composition of the invention, particularly the chromium or chromium compound which is present in a proportionally much lower amount, and to the attainment of dose uniformity of the active ingredients, particularly the chromium compound, in the composition. This makes it easy for each dosage unit to contain the stated amount of each active substance and, likewise, allows preparing the composition in fewer steps, which is highly significant on an industrial level.

In another preferred embodiment, if the composition contains vitamin D, preferably vitamin D3, more preferably Dry Vitamin D3 100 CWS, said vitamin has a particle size between 75 and 850 µm, preferably at least 90% of the particles has a (volume weighted) particle size between 100 and 500 µm.

Likewise, if the composition contains vitamin D, preferably vitamin D3, more preferably Dry Vitamin D3 100 CWS, said vitamin has a particle size distribution with the following characteristics:
- D90 equal to or less than 400 µm, such as between 400 and 330 µm, preferably between 380 and 350 µm, particularly about 365 µm.
- D50 equal to or less than 250 µm, such as between 220 and 250 µm, preferably between 230 and 240 µm, particularly about 235 µm.
- D10 equal to or less than 200 µm, such as between 200 and 100 µm, preferably between 170 and 130 µm, particularly about 151 µm.
- D[4,3] between 200 and 300 µm, preferably between 230 and 270 µm, more preferably about 249 µm.

In a preferred embodiment, the composition of the present invention comprises:
- between 600 and 6,000 mg of inositol, preferably myo-inositol or a mixture containing myo-inositol in a majority percentage by weight,
- between 2.5 and 250 µg of chromium, preferably chromium III, preferably comprised in a compound, more preferably between 20 and 2,000 µg of a chromium III compound which is chromium picolinate,
- optionally, between 5 and 100 µg of vitamin D, preferably vitamin D3,

wherein the weight ratio between chromium, preferably chromium III, and inositol, preferably myo-inositol, is comprised between 1/100,000 and 1/800,000, preferably between 1/104,000 and 1/720,000, preferably between 1/104,000 and 1/600,000, of chromium/inositol,
and wherein the ratio between the mean particle diameters D[4,3] of the inositol, preferably myo-inositol, or a mixture containing myo-inositol in a majority percentage by weight, and of the chromium, preferably the chromium compound, more preferably chromium picolinate, is between 3/1 and 1/1, preferably between 2.5/1 and 1.5/1, of inositol/chromium.

Alternatively, when it contains chromium picolinate, the weight ratio between chromium picolinate and myo-inositol is comprised between 1/12,500 and 1/100,000, preferably between 1/13,000 and 1/90,000, of chromium picolinate/inositol,
and the ratio between the mean particle diameters D[4,3] of myo-inositol and chromium picolinate is between 3/1 and 1/1, preferably between 2.5/1 and 1.5/1, of myo-inositol/chromium picolinate.

Alternatively, when it contains chromium picolinate, the weight ratio between chromium picolinate and myo-inositol is comprised between 1/12,500 and 1/100,000, preferably between 1/13,000 and 1/90,000, of chromium picolinate/inositol,
and myo-inositol has a particle size distribution D90 equal to or less than 150 µm, such as between 150 and 70 µm, preferably between 100 and 120, more preferably between 105 and 115 µm, particularly about 111 µm, and
chromium picolinate has a particle size distribution D90 equal to or less than 75 µm, such as between 75 and 35 µm, preferably between 65 and 45 µm, particularly about 53 µm.

In a particularly preferred embodiment, the composition comprises:
- between 1,000 and 5,000 mg of inositol, preferably myo-inositol or a mixture of inositol stereoisomers which contains myo-inositol in a majority percentage by weight,
- between 2.5 and 250 µg of chromium, preferably chromium III, preferably comprised in a compound, more preferably between 20 and 2,000 µg of a chromium III compound which is chromium picolinate,
- optionally, between 5 and 100 µg of vitamin D (corresponding to between 200 and 4,000 IU), preferably vitamin D3,

wherein the weight ratio between chromium, or chromium III, and inositol, or myo-inositol, is comprised between 1/100,000 and 1/800,000, preferably between 1/104,000 and 1/720,000, preferably between 1/104,000 and 1/600,000, more preferably between 1/120,000 and 1/720,000, between 1/160,000 and 1/600,000, between 1/184,000 and 1/560,000, between 1/200,000 and 1/504,000, of chromium/inositol,
and wherein the ratio between the mean particle diameters D[4,3] of the inositol, preferably myo-inositol, and of the chromium, preferably the chromium compound, more preferably chromium picolinate, is between 3/1 and 1/1, preferably between 2.5/1 and 1.5/1, of inositol/chromium.

Alternatively, when it contains chromium picolinate, the weight ratio between chromium picolinate and myo-inositol is comprised between 1/12,500 and 1/100,000, preferably between 1/13,000 and 1/90,000, more preferably between 1/15,000 and 1/90,000, even more preferably between 1/20,000 and 1/75,000, between 1/23,000 and 1/70,000, between 1/25,000 and 1/63,000, of chromium picolinate/myo-inositol,
and the ratio between the mean particle diameters D[4,3] of myo-inositol and chromium picolinate is between 3/1 and 1/1, preferably between 2.5/1 and 1.5/1, of myo-inositol/chromium picolinate.

Alternatively, when it contains chromium picolinate, the weight ratio between chromium picolinate and myo-inositol is comprised between 1/12,500 and 1/100,000, preferably between 1/13,000 and 1/90,000, more preferably between 1/15,000 and 1/90,000, even more preferably between 1/20,000 and 1/75,000, between 1/23,000 and 1/70,000, between 1/25,000 and 1/63,000, of chromium picolinate/myo-inositol,
and myo-inositol has a particle size distribution D90 equal to or less than 150 µm, such as between 150 and 70 µm, preferably between 100 and 120, more preferably between 105 and 115 µm, particularly about 111 µm, and
chromium picolinate has a particle size distribution D90 equal to or less than 75 µm, such as between 75 and 35 µm, preferably between 65 and 45 µm, particularly about 53 µm.

In another particularly preferred embodiment, the composition comprises:
- between 3,000 and 5,000 mg of inositol, preferably myo-inositol or a mixture of inositol stereoisomers which contains myo-inositol in a majority percentage by weight,
- between 6 and 125 µg of chromium, preferably chromium III, more preferably between 50 and 1,000 µg of a chromium III compound which is chromium picolinate,
- optionally, between 15 and 100 µg of vitamin D, preferably vitamin D3,

wherein the weight ratio between chromium, or chromium III, and inositol, or myo-inositol, is comprised between 1/120,000 and 1/720,000, preferably between 1/160,000 and 1/600,000, more preferably between 1/184,000 and 1/560,000, even more preferably between 1/200,000 and 1/504,000 of chromium/inositol,
and wherein the ratio between the mean particle diameters D[4,3] of the inositol, preferably myo-inositol, and of the chromium, preferably the chromium compound, more preferably chromium picolinate, is between 3/1 and 1/1, preferably between 2.5/1 and 1.5/1, of inositol/chromium.

Alternatively, when it contains chromium picolinate, the weight ratio between chromium picolinate and myo-inositol is comprised between 1/15,000 and 1/90,000, preferably between 1/20,000 and 1/75,000, more preferably between 1/23,000 and 1/70,000, even more preferably between 1/25,000 and 1/63,000, of chromium picolinate/myo-inositol,
and the ratio between the mean particle diameters D[4,3] of myo-inositol and chromium picolinate is between 3/1 and 1/1, preferably between 2.5/1 and 1.5/1, of myo-inositol/chromium picolinate.

Alternatively, when it contains chromium picolinate, the weight ratio between chromium picolinate and myo-inositol is comprised between 1/15,000 and 1/90,000, preferably between 1/20,000 and 1/75,000, more preferably between 1/23,000 and 1/70,000, even more preferably between 1/25,000 and 1/63,000, of chromium picolinate/myo-inositol,
and myo-inositol has a particle size distribution D90 equal to or less than 150 µm, such as between 150 and 70 µm, preferably between 100 and 120, more preferably between 105 and 115 µm, particularly about 111 µm, and
chromium picolinate has a particle size distribution D90 equal to or less than 75 µm, such as between 75 and 35 µm, preferably between 65 and 45 µm, particularly about 53 µm.

In a particularly preferred embodiment, the composition comprises:
- between 3,500 and 4,500 mg of inositol, preferably myo-inositol or a mixture of inositol stereoisomers which contains myo-inositol in a majority percentage by weight,
- between 8.5 and 62.5 µg of chromium, preferably chromium III, more preferably between 70 and 500 µg of chromium picolinate,
- optionally, between 20 and 50 µg of vitamin D, preferably vitamin D3,

wherein the weight ratio between chromium, or chromium III, and inositol, or myo-inositol, is comprised between 1/184,000 and 1/560,000, preferably between 1/200,000 and 1/504,000, particularly 1/400,000, of chromium/inositol,
and wherein the ratio between the mean particle diameters D[4,3] of the inositol, preferably myo-inositol, and of the chromium, preferably the chromium compound, more preferably chromium picolinate, is between 3/1 and 1/1, preferably between 2.5/1 and 1.5/1, of inositol/chromium.

Alternatively, when it contains chromium picolinate, the weight ratio between chromium picolinate and myo-inositol is comprised between 1/20,000 and 1/75,000, preferably between 1/23,000 and 1/70,000, more preferably between 1/25,000 and 1/63,000, particularly about 1/50,000, of chromium picolinate/myo-inositol, and the ratio between the mean particle diameters D[4,3] of myo-inositol and chromium picolinate is between 3/1 and 1/1, preferably between 2.5/1 and 1.5/1, of myo-inositol/chromium picolinate.

Alternatively, when it contains chromium picolinate, the weight ratio between chromium picolinate and myo-inositol is comprised between 1/20,000 and 1/75,000, preferably between 1/23,000 and 1/70,000, more preferably between 1/25,000 and 1/63,000, particularly about 1/50,000, of chromium picolinate/myo-inositol,
and myo-inositol has a particle size distribution D90 equal to or less than 150 µm, such as between 150 and 70 µm, preferably between 100 and 120, more preferably between 105 and 115 µm, particularly about 111 µm, and
chromium picolinate has a particle size distribution D90 equal to or less than 75 µm, such as between 75 and 35 µm, preferably between 65 and 45 µm, particularly about 53 µm.

In another particularly preferred embodiment, the composition of the invention comprises:
- between 1,000 and 3,000 mg of inositol, preferably myo-inositol or a mixture of inositol stereoisomers which contains myo-inositol in a majority percentage by weight,
- between 2.5 and 62.5 µg of chromium, preferably chromium III, more preferably between 20 and 500 µg of chromium picolinate,
- optionally, between 5 and 50 µg of vitamin D, preferably vitamin D3,

wherein the weight ratio between chromium, or chromium III, and inositol, or myo-inositol, is comprised between 1/120,000 and 1/800,000, preferably between 1/160,000 and 1/600,000, more preferably between 1/184,000 and 1/560,000, even more preferably between 1/200,000 and 1/504,000, of chromium/inositol,
and wherein the ratio between the mean particle diameters D[4,3] of the inositol, preferably myo-inositol, and of the chromium, preferably the chromium compound, more preferably chromium picolinate, is between 3/1 and 1/1, preferably between 2.5/1 and 1.5/1, of inositol/chromium.

Alternatively, when it contains chromium picolinate, the weight ratio between chromium picolinate and myo-inositol is comprised between 1/15,000 and 1/100,000, preferably between 1/20,000 and 1/75,000, more preferably between 1/23,000 and 1/70,000, even more preferably between 1/25,000 and 1/63,000, of chromium picolinate/myo-inositol,
and the ratio between the mean particle diameters D[4,3] of myo-inositol and chromium picolinate is between 3/1 and 1/1, preferably between 2.5/1 and 1.5/1, of myo-inositol/chromium picolinate.

Alternatively, when it contains chromium picolinate, the weight ratio between chromium picolinate and myo-inositol is comprised between 1/15,000 and 1/100,000, preferably between 1/20,000 and 1/75,000, more preferably between 1/23,000 and 1/70,000, even more preferably between 1/25,000 and 1/63,000, of chromium picolinate/myo-inositol,
and myo-inositol has a particle size distribution D90 equal to or less than 150 µm, such as between 150 and 70 µm, preferably between 100 and 120, more preferably between 105 and 115 µm, particularly about 111 µm, and
chromium picolinate has a particle size distribution D90 equal to or less than 75 µm, such as between 75 and 35 µm, preferably between 65 and 45 µm, particularly about 53 µm.

In a particularly preferred embodiment, the composition comprises:
- between 1,500 and 2,500 mg of inositol, preferably myo-inositol or a mixture of inositol stereoisomers which contains myo-inositol in a majority percentage by weight,
- between 3.1 and 31.0 µg of chromium, preferably chromium III, more preferably between 25 and 250 µg of a chromium III compound which is chromium picolinate,
- optionally, between 10 and 25 µg of vitamin D, particularly of vitamin D3,

wherein the weight ratio between chromium, or chromium III, and inositol, or myo-inositol, is comprised between 1/184,000 and 1/560,000, preferably between 1/200,000 and 1/504,000, particularly 1/400,000, of chromium/inositol,
and wherein the ratio between the mean particle diameters D[4,3] of the inositol, preferably myo-inositol, and of the chromium, preferably the chromium compound, more preferably chromium picolinate, is between 3/1 and 1/1, preferably between 2.5/1 and 1.5/1, of inositol/chromium.

Alternatively, when it contains chromium picolinate, the weight ratio between chromium picolinate and myo-inositol is comprised between 1/20,000 and 1/75,000, preferably between 1/23,000 and 1/70,000, more preferably between 1/25,000 and 1/63,000, particularly about 1/50,000, of chromium picolinate/myo-inositol,
and the ratio between the mean particle diameters D[4,3] of myo-inositol and chromium picolinate is between 3/1 and 1/1, preferably between 2.5/1 and 1.5/1, of myo-inositol/chromium picolinate.

Alternatively, when it contains chromium picolinate, the weight ratio between chromium picolinate and myo-inositol is comprised between 1/20,000 and 1/75,000, preferably between 1/23,000 and 1/70,000, more preferably between 1/25,000 and 1/63,000, particularly about 1/50,000, of chromium picolinate/myo-inositol,
and myo-inositol has a particle size distribution D90 equal to or less than 150 µm, such as between 150 and 70 µm, preferably between 100 and 120, more preferably between 105 and 115 µm, particularly about 111 µm, and
chromium picolinate has a particle size distribution D90 equal to or less than 75 µm, such as between 75 and 35 µm, preferably between 65 and 45 µm, particularly about 53 µm.

In another particularly preferred embodiment, the composition comprises:
- between 600 and 1,500 mg of inositol, preferably myo-inositol,
- between 1.2 and 12.5 µg of chromium, preferably chromium III, preferably comprised in a compound, more preferably between 10 and 100 µg of chromium picolinate,
- optionally, between 5 and 50 µg of vitamin D, preferably vitamin D3,

wherein the weight ratio between chromium, or chromium III, and inositol, or myo-inositol, is comprised between 1/104,000 and 1/600,000, particularly between 1/120,000 and 1/400,000, of chromium/inositol,
and wherein the ratio between the mean particle diameters D[4,3] of the inositol, preferably myo-inositol, and of the chromium, preferably the chromium compound, more preferably chromium picolinate, is between 3/1 and 1/1, preferably between 2.5/1 and 1.5/1, of inositol/chromium.

Alternatively, when it contains chromium picolinate, the weight ratio between chromium picolinate and myo-inositol is comprised between 1/13,000 and 1/75,000, preferably between 1/15,000 and 1/50,000, of chromium picolinate/myo-inositol, and the ratio between the mean particle diameters D[4,3] of myo-inositol and chromium picolinate is between 3/1 and 1/1, preferably between 2.5/1 and 1.5/1, of myo-inositol/chromium picolinate.

Alternatively, when it contains chromium picolinate, the weight ratio between chromium picolinate and myo-inositol is comprised between 1/13,000 and 1/75,000, preferably between 1/15,000 and 1/50,000, of chromium picolinate/myo-inositol,
and myo-inositol has a particle size distribution D90 equal to or less than 150 µm, such as between 150 and 70 µm, preferably between 100 and 120, more preferably between 105 and 115 µm, particularly about 111 µm, and
chromium picolinate has a particle size distribution D90 equal to or less than 75 µm, such as between 75 and 35 µm, preferably between 65 and 45 µm, particularly about 53 µm.

The composition of the invention can be formulated in any solid pharmaceutical form for oral administration such as capsules, tablets, granulates or powder. Preferably, the composition is in the form of tablets, powder or granules (dispensed in sachets or dispersed in a pharmaceutically acceptable liquid).

In a particular embodiment, this composition is in the form of a granulate or powder contained in a sachet or in the form of a granulate or powder dispersed in a pharmaceutically acceptable liquid such as water, preferably for oral administration.

The composition of the invention may further comprise non-active ingredients, such as pharmaceutically or nutritionally acceptable excipients, known to one skilled in the art. Particularly, it may contain one or more diluents, binders, disintegrants, lubricants, glidants, sweeteners, flavourings, aromas and/or effervescent agents.

Preferably, the composition contains at least one glidant selected from the group consisting of silica derivatives (for example, silicon dioxide, hydrated silica, hydrophilic or hydrophobic precipitated silica, hydrophilic fumed silica, colloidal silica, anhydrous colloidal silica, aluminium silicate, magnesium silicate, magnesium aluminium silicate, sodium silicate, sodium aluminium silicate, calcium aluminium silicate, calcium silicate (monocalcium, dicalcium or tricalcium, anhydrous or hydrated), silicic acid), talc, stearic acid and its calcium and/or magnesium salts, sodium stearyl fumarate, starch and mixtures thereof. More preferably it contains colloidal silicon dioxide.

The composition may further contain one or more disintegrants (selected, for example, from the group consisting of hydroxypropyl cellulose [L-HPC], crospovidone, sodium croscarmellose, calcium and sodium alginates, sodium starch glycolate and other derivatised starches (for example, corn, potato, rice, wheat, cassava and tuber starch, pregelatinised starch, hydrolysed starch, modified starch, and mixtures thereof), one or more binders (selected, for example, from the group consisting of hydroxypropyl cellulose [L-HPC], povidone and derivatives thereof (for example, crospovidone, vinylpyrrolidone, polyvinylpyrrolidone and salts thereof, polyvinylpolypyrrolidone), cellulose and derivatives thereof (for example, crystalline cellulose, microcrystalline cellulose, cross-linked sodium cellulose, carboxymethylcellulose, sodium carboxymethylcellulose, cross-linked sodium carboxymethylcellulose, methylcellulose, ethylcellulose, hydroxypropylmethylcellulose), maltodextrin and other cyclodextrins, starch and derivatives thereof (for example, corn, potato, rice, wheat, cassava and tuber starch, pregelatinised starch, sodium starch glycolate, hydrolysed starch, modified starch, and mixtures thereof) and mixtures thereof, one or more diluents (selected, for example, from the group consisting of microcrystalline celluloses, starches and derivatives thereof (for example, corn, potato, rice, wheat, cassava and tuber starch, pregelatinised starch, sodium starch glycolate, hydrolysed starch, modified starch, and mixtures thereof), sugars and derivatives thereof (for example, glucose, mannose, fructose, galactose, lactose, sucrose, maltose, sucralose, isomaltose, mannitol, maltitol, lactitol, sorbitol, galactitol, xylitol, erythritol, cyclamate, and mixtures thereof).

The use of the excipients described above is particularly suitable when the composition is in the form of a capsule or tablet for oral administration.

When the composition is in the form of powder to be dispensed in sachets, adding one or more glidants often suffices.

The composition of the invention can be a drug or a food supplement.

In the context of the present invention, the terms "food supplement", "nutritional supplement", "dietary supplement" or "nutraceuticals" are equivalent and refer to products the purpose of which is to supplement the normal diet or to make up for certain dietary deficiency. They include concentrated sources of nutrients or other substances with a nutritional or physiological effect, in a simple or combined form, which are marketed in oral dosage forms such as tablets, pills, capsules, granules or powder.

In a second aspect, the present invention relates to the use of a composition according to the first aspect of the invention as a food supplement, particularly for reducing or preventing elevated plasma glucose levels or maintaining plasma glucose levels within normal values.

In a second alternative aspect, the present invention relates to a composition according to the first aspect of the invention, for use thereof in medicine.

More particularly, the present invention relates to a composition according to the first aspect of the invention, for use in the treatment and/or the prevention of a metabolic disease and/or disorder characterised by elevated plasma glucose levels, for example, diabetes mellitus.

In a preferred embodiment, the diabetes mellitus is gestational diabetes mellitus.

In another aspect, the invention relates to a method for the treatment and/or the prevention of a metabolic disease and/or disorder characterised by elevated plasma glucose levels, said method comprises administering to a patient in need of said treatment a composition according to the first aspect of the invention. Preferably, the metabolic disease and/or disorder characterised by elevated plasma glucose levels is diabetes mellitus, more preferably gestational diabetes mellitus.

In another aspect, the invention relates to the use of a composition according to the first aspect of the invention, in the preparation of a drug for the treatment and/or the prevention of a metabolic disease and/or disorder characterised by elevated plasma glucose levels. Preferably, the metabolic disease and/or disorder characterised by elevated plasma glucose levels is diabetes mellitus, more preferably gestational diabetes mellitus.

In an additional aspect, the present invention relates to the method of manufacturing a composition according to the first aspect of this invention. All the definitions and preferences indicated in the first aspect of the invention also apply to the third aspect of the invention.

In a preferred embodiment, the method of manufacturing comprises the following steps:
1. optionally, if the composition contains vitamin D, first mixing the entire amount of vitamin D and chromium, preferably a chromium compound,
2. preparing a first premixture (P1) by mixing between 1% and 11% by weight, preferably between 2% and 10%, more preferably between 2.5% and 7.5% by weight of the total amount of inositol with part, preferably with at least 20% by weight, more preferably with at least 30%, even more preferably with at least 40%, particularly with half, of the total amount of chromium, preferably the chromium compound or, if step 1 was performed, with part, preferably with at least 20% by weight, more preferably with at least 30%, even more preferably with at least 40%, particularly with half, of the total amount of the mixture of vitamin D and chromium, preferably the chromium compound, of step 1,
3. preparing a second premixture (P2) by mixing between 1% and 11% by weight, preferably between 2% and 10%, more preferably between 2.5% and 7.5% by weight of the total amount of inositol with the remaining amount of chromium, preferably the chromium compound or, if step 1 was performed, with the remaining amount of the mixture of vitamin D and chromium, preferably the chromium compound, of step 1,
4. adding into a mixing container and mixing:
   a. at least 30% by weight of the amount of inositol that remains after steps 2 and 3,
   b. the first premixture of step 2,
   c. the second premixture of step 3,
5. optionally, adding the amount of inositol that remains after step 4 and mixing,
6. optionally, adding the excipient or excipients and mixing.

In the context of the present invention, the total amount of an ingredient when mentioned refers to the total of that amount that is to be comprised in the composition manufactured according to the first aspect of the invention.

In a particular embodiment, between 30% and 50% by weight of the amount of inositol that remains after steps 2 and 3 are added in step 4.

In a more particular embodiment, 70 % of the amount of inositol that remains after steps 2 and 3 are added in step 4.

In another particularly preferred embodiment, step 5 is not performed and the total amount of inositol that remains after steps 2 and 3 is added and mixed in step 4.a.

In a preferred embodiment, the composition comprises chromium picolinate and myo-inositol, and the method of manufacturing comprises the following steps:
1. optionally, if the composition contains vitamin D, first mixing the entire amount of vitamin D and chromium picolinate,
2. preparing a first premixture (P1) by mixing between 2% and 10% by weight of the total amount of myo-inositol, preferably between 2.5% and 7.5%, more preferably between 2.5% and 5%, even more preferably about 2.5% of the total amount of myo-inositol with part, preferably with at least 20% by weight, particularly with half, of the total amount of chromium picolinate or, if step 1 was performed, with part, preferably with at least 20% by weight, particularly with half, of the total amount of the mixture of vitamin D and chromium picolinate of step 1,
3. preparing a second premixture (P2) by mixing between 2% and 10% by weight of the total amount of myo-inositol, preferably between 2.5% and 7.5%, more preferably between 2.5% and 5%, even more preferably about another 2.5% of the total amount of myo-inositol with the remaining amount of chromium picolinate or, if step 1 was performed, with the remaining amount of the mixture of vitamin D and chromium picolinate of step 1,
4. adding into the mixing container the total amount of myo-inositol and the premixtures in the following order and mixing:
   a. at least 30% by weight of the amount of myo-inositol that remains after steps 2 and 3,
   b. the first premixture of step 2,
   c. the second premixture of step 3,
   d. the amount of myo-inositol that remains after step 4a., and mixing,
5. optionally, adding the excipient or excipients and mixing.

In another particular embodiment, step 5 is not performed and the total amount of myo-inositol that remains after steps 2 and 3 is added and mixed in step 4.a.

The premixtures of the method of the present invention allow diluting the active ingredients with a part of the excipients and thereby achieving a homogenous mixing in that fraction of the formulation. These premixtures are then mixed with the rest of the excipients to thereby favour obtaining formulations with a uniform content of active ingredients, particularly of the active ingredient or ingredients present in a relatively very low amount with respect to the amount of the other components.

Surprisingly, this method of preparation of the present invention allows obtaining the composition of the present invention with a uniform content of the components in fewer steps (all the remaining amount of inositol can be added in a single step 4.), despite the chromium compound, and optionally vitamin D, being present in a proportionally much lower amount with respect to inositol. Achieving a uniform mixture in fewer steps is highly relevant on an industrial level since it allows reducing products times and costs.

The use of inositol, preferably myo-inositol or a mixture of inositol stereoisomers which contains myo-inositol in a majority percentage by weight, and of chromium or chromium compound, preferably chromium picolinate, with a similar particle size distribution, as described in the first aspect of the invention, is believed to favour the attainment of compositions with a uniform content of the active ingredients in fewer steps. Content uniformity can be verified by means of any conventional method, particularly through a measurement as described in the Examples.

In additional embodiments, any instance of the term "comprises" or "contains" described herein can be interpreted independently as "consists of".

### EXAMPLES

### Formulations according to the invention

### 1. Formulation in sachets (1/day)

| Ingredient | mg/sachet | % (w/w) |
|---|---|---|
| Myo-inositol | 4000 | 99.350 |
| Vitamin D3 CWS 100* | 10 | 0.248 |
| Chromium picolinate** | 0.08 | 0.002 |
| Colloidal silica | 16 | 0.400 |
| Total | 4026.08 | 100.00 |

| | | |
|---|---|---|
| * equivalent to 25 µg (1000 IU) of vitamin D3 ** equivalent to 10 µg of chromium | | |

### 2. Formulation in tablets (2 or 3/day)

| Ingredient | mg/tablet | % (g/100 g) |
|---|---|---|
| Myo-inositol | 700.00 | 71.79 |
| Chromium picolinate | 0.03 | 0.003 |
| CMC Vivapur 102 | 115.00 | 11.79 |
| CMC Vivapur 200 | 117.17 | 12.03 |
| L-HPC LH-11 | 12.80 | 1.31 |
| Aerosil 200 Pharma | 10.00 | 1.03 |
| Magnesium stearate | 20.00 | 2.05 |
| Total | 975.00 | 100 |

### 3. Formulation in tablets (2/day)

| Components | mg/tablet | % (g/100 g) |
|---|---|---|
| Myo-inositol | 800.00 | 78.82 |
| Chromium picolinate | 0.04 | 0.004 |
| Silicon dioxide | 19.00 | 1.87 |
| Sodium croscarmellose | 15.00 | 1.48 |
| Povidone K30 | 23.00 | 2.27 |
| Crospovidone | 28.00 | 2.76 |
| Hydroxypropyl cellulose | 71.90 | 7.08 |
| Microcrystalline cellulose | 35.00 | 3.45 |
| Magnesium stearate | 8.00 | 0.79 |
| Hydroxypropylmethylcellulose* | 12.00 | 1.18 |
| Microcrystalline cellulose* | 1.50 | 0.15 |
| Stearic acid* | 1.50 | 0.15 |
| TOTAL | 1014.94 | 100 |

| | | |
|---|---|---|
| * Transparent coating components (Sepifilm LP 010) | | |

### Analysis of the homogeneity of the active ingredients in the finished product

In order to evaluate the correct homogeneity of the active ingredients in the finished product, samples are taken at different mixing times and different product discharge points upon the completion of mixing or at different points of the container where the mixture is discharged. The methods of analysis for each of the minority active ingredients (vitamin D and chromium picolinate) are described below:

### Vitamin D identification and content

### Vitamin D identification in HPLC

Vitamin D identification is performed by HPLC, by retention time and by UV spectrum:
- Identification 1 (HPLC tr.): The difference between the retention time of vitamin D in the sample chromatogram and in the reference standard should be ≤ 2.5%.
- Identification 2 (UV spectrometry): The spectrum of vitamin D in the sample chromatogram should have around the same maximum (about 266 nm) as in the standard chromatogram. This identification is performed with high-performance liquid chromatography (HPLC) with a diode array detector (DAD).

### Vitamin D content in HPLC

| CHROMATOGRAPHIC CONDITIONS | |
|---|---|
| Equipment | HPLC Agilent with DAD or equivalent |
| Column | Phenomenex Kinetex F5 2.6 µm 100x4.6 mm |
| Precolumn | Phenomenex F5 |
| Wavelength | 267 nm |
| Column temperature | 25ºC |
| Flow | 1.0 ml/min |
| Injection volume | 10 µL |
| Analysis time | 15 min |
| Approximate retention time of vit. D3 | around 7 min |
| Mobile phase | 0.1% formic acid in MeOH/0.1% formic acid in water, 85:15 |

### Specifications

### Bulk and bulk specification during wait period: 24,814 IU/100 g (80-130%): 19,851-32,258 IU/100 g Specification in finished product: 1000.0 IU/sachet (80-130%): 800.0 - 1300.0 IU/sachet.

### Chromium picolinate identification and content

### Chromium picolinate identification in HPLC

Chromium picolinate identification is performed by HPLC, by retention time and by UV spectrum:
- Identification 1 (HPLC tr.): The difference between the retention time of chromium picolinate in the sample chromatogram and in the reference standard must be ≤ 2.5%.
- Identification 2 (UV spectrometry): The spectrum of chromium picolinate in the sample chromatogram should have around the same maximums as in the standard chromatogram. This identification is performed with HPLC DAD.

### Chromium picolinate content in HPLC

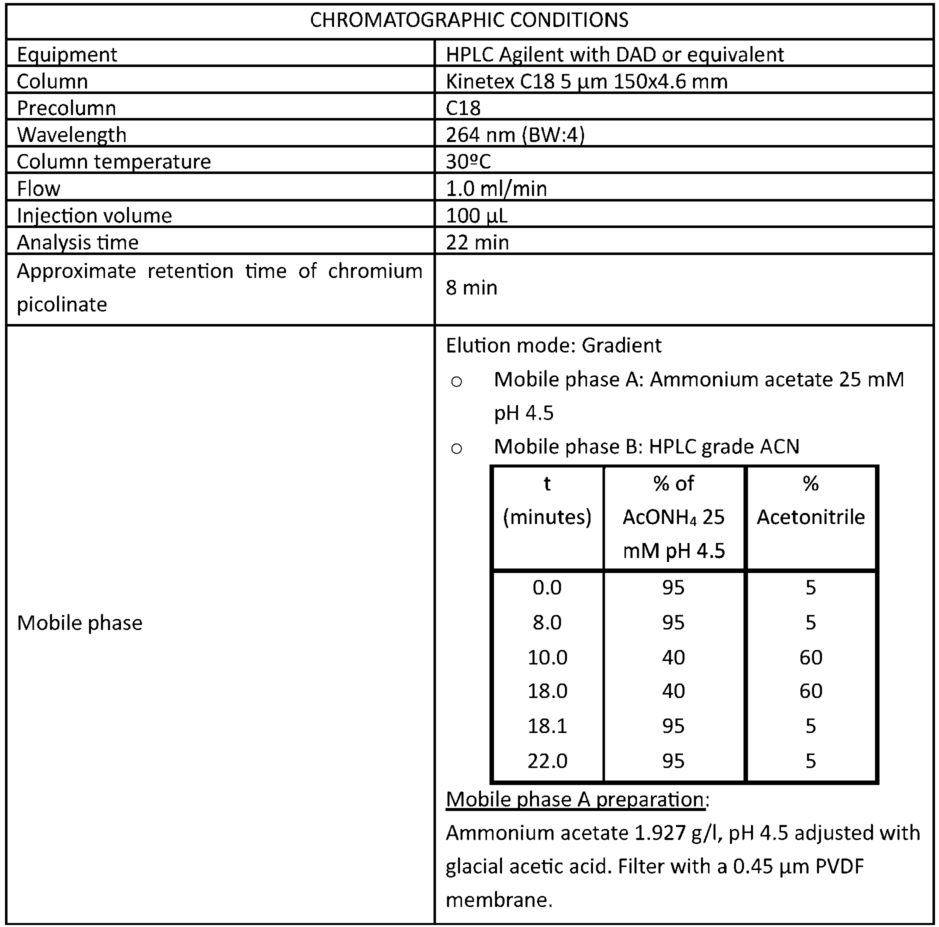

### Specifications

Bulk and bulk specification during wait period: 1996 µg/100 g (75-150%) = 1497 - 2994 µg/100 g

Specification in finished product: 80.45 µg/sachet (75-150%) = 60.34 - 120.68 µg/sachet

## Claims

1. A composition comprising:
- between 600 and 6,000 mg of inositol;
- between 2.5 and 250 µg of chromium, preferably in the form of a pharmaceutically acceptable compound comprising between 2.5 and 250 µg of chromium; and
- optionally, between 5 and 100 µg of vitamin D.

2. The composition according to claim 1, wherein the chromium/inositol weight ratio is comprised between 1/100,000 and 1/800,000; preferably between 1/104,000 and 1/720,000.

3. The composition according to any of claims 1 or 2, comprising:
- between 3,000 and 5,000 mg of inositol, which is myo-inositol or a mixture of inositol stereoisomers which contains myo-inositol in a majority percentage by weight,
- between 50 and 1,000 µg of a chromium compound, which is chromium picolinate,
- optionally, between 15 and 100 µg of vitamin D.

4. The composition according to claim 3, wherein the weight ratio between chromium picolinate and myo-inositol is comprised between 1/15,000 and 1/90,000, preferably between 1/20,000 and 1/75,000, of chromium picolinate/myo-inositol.

5. The composition according to any of claims 1 or 2, comprising:
- between 1,000 and 3,000 mg of inositol, which is myo-inositol or a mixture of inositol stereoisomers which contains myo-inositol in a majority percentage by weight,
- between 20 and 500 µg of a chromium compound, which is chromium picolinate,
- optionally, between 5 and 50 µg of vitamin D.

6. The composition according to claim 5, wherein the weight ratio between chromium picolinate and myo-inositol is comprised between 1/15,000 and 1/100,000, preferably between 1/20,000 and 1/75,000, of chromium picolinate/myo-inositol.

7. The composition according to any of claims 3 to 6, in the form of a granulate or powder, for oral administration, and contained in a sachet.

8. The composition according to any of claims 3 to 6, in the form of a granulate or powder dispersed in a pharmaceutically acceptable liquid, for oral administration.

9. The composition according to any of claims 3 to 6, wherein the weight ratio between chromium picolinate and myo-inositol is comprised between 1/23,000 and 1/70,000, preferably between 1/25,000 and 1/70,000.

10. The composition according to any of claims 1 or 2, comprising:
- between 600 and 1,500 mg of inositol, which is myo-inositol or a mixture of inositol stereoisomers which contains myo-inositol in a majority percentage by weight,
- between 10 and 100 µg of a chromium compound, which is chromium picolinate,
- optionally, between 5 and 50 µg of vitamin D.

11. The composition according to claim 10, wherein the weight ratio between chromium picolinate and myo-inositol is comprised between 1/13,000 and 1/75,000, preferably between 1/15,000 and 1/50,000.

12. The composition according to claims 10 or 11, in the form of a capsule or tablet, for oral administration.

13. The composition according to any of claims 10 to 12, wherein the weight ratio between chromium picolinate and myo-inositol is comprised between 1/20,000 and 1/50,000.

14. The composition according to any of the preceding claims, wherein the inositol has a particle size distribution D90 equal to or less than 150 µm, and the chromium compound has a particle size distribution D90 equal to or less than 75 µm.

15. The composition according to claim 1, comprising:
- between 600 and 6,000 mg of inositol, preferably myo-inositol or a mixture containing myo-inositol in a majority percentage by weight,
- between 2.5 and 250 µg of chromium, preferably chromium III, preferably comprised in a compound, more preferably between 20 and 2,000 µg of a chromium III compound which is chromium picolinate,
- optionally, between 5 and 100 µg of vitamin D, preferably vitamin D3,
wherein the weight ratio between chromium, preferably chromium III, and inositol, preferably myo-inositol, is comprised between 1/100,000 and 1/800,000, preferably between 1/104,000 and 1/720,000, more preferably between 1/120,000 and 1/720,000, between 1/160,000 and 1/600,000, between 1/200,000 and 1/600,000, of chromium/inositol,
and wherein the ratio between the mean particle diameters D[4,3] of the inositol, preferably myo-inositol, or a mixture containing myo-inositol in a majority percentage by weight, and of the chromium, preferably the chromium compound, more preferably chromium picolinate, is between 3/1 and 1/1, preferably between 2.5/1 and 1.5/1.

16. The composition according to any of the preceding claims, for use thereof in medicine.

17. The composition for use thereof according to claim 16, for use in the treatment and/or the prevention of a metabolic disease and/or disorder **characterised by** the existence of elevated plasma glucose levels.

18. The composition for use thereof according to claim 16, for use in the treatment and/or the prevention of diabetes mellitus.

19. The composition for use thereof according to claim 16, for use in the treatment and/or the prevention of gestational diabetes mellitus.

20. A food supplement comprising the composition according to any of claims 1 to 15.

21. A method of manufacturing the composition of any of claims 1 to 15, comprising the following steps:
1. optionally, if the composition contains vitamin D, first mixing the entire amount of vitamin D and the chromium or chromium compound,
2. preparing a first premixture by mixing between 1% and 11% by weight of the total amount of inositol with part of the total amount of chromium or chromium compound or, if step 1 was performed, with part of the mixture of vitamin D and chromium or chromium compound of step 1,
3. preparing a second premixture by mixing between 1% and 11% by weight of the total amount of inositol with the remaining amount of chromium or chromium compound or, if step 1 was performed, with the remaining amount of the mixture of vitamin D and chromium or chromium compound of step 1,
4. adding into a mixing container and mixing:
a. at least 30% by weight of the amount of inositol that remains after steps 2 and 3,
b. the first premixture of step 2,
c. the second premixture of step 3,
5. optionally adding the amount of inositol that remains after step 4, and mixing,
6. optionally adding the excipient or excipients and mixing.
